Europäisches Patentamt

European Patent Office

Office européen des brevets

(11) Numéro de publication: **0 308 279**
**A1**

## (12) DEMANDE DE BREVET EUROPEEN

(21) Numéro de dépôt: 88402048.8

(22) Date de dépôt: 05.08.88

(51) Int. Cl.4: **C 12 P 19/04**
C 08 B 37/00, A 61 K 31/715
//(C12P19/04,C12R1:19,1:22)

(30) Priorité: 08.09.87 FR 8712407

(43) Date de publication de la demande:
22.03.89 Bulletin 89/12

(84) Etats contractants désignés:
AT BE CH DE ES FR GB GR IT LI LU NL SE

(71) Demandeur: **LIPHA, LYONNAISE INDUSTRIELLE PHARMACEUTIQUE**
**34, rue Saint Romain Boîte Postale 8481**
**F-69359 Lyon Cedex 08 (FR)**

(72) Inventeur: **Carel, Serge**
**Chemin des Ecoliers 5**
**CH-1816 Chailly-Montreux (CH)**

**Page, Yves-Marie**
**Avenue des Bosquets de Julie 66**
**CH-1815 (CH)**

**Vanderhoven, Christine**
**Route de Taillepied 36**
**CH-1095 Lutry (CH)**

**Murray, Norman**
**Chemin du Devin 53**
**CH-1012 Lausanne (CH)**

**Monsigny, Michel**
**Rue des Bouvreuils 341**
**F-45590 Saint-Cyr-en-Val (FR)**

**Delmotte, Francis**
**Rue Philippe-de-Commynes**
**F-45160 Olivet (FR)**

**Roche, Anne-Claude**
**rue de Saint-mesmin 360**
**F-45750 Saint-Pryve (FR)**

**Petit, Claire**
**Rue de l'Ecu d'Or**
**F-45000 Orléans (FR)**

(74) Mandataire: **Bouton Neuvy, Liliane et al**
**L'Air liquide, Société Anonyme pour L'Etude et L'Exploitation des Procédés Georges Claude 75, Quai d'Orsay**
**F-75321 Paris Cédex 07 (FR)**

(54) Nouveaux polysaccharides hydrosolubles, leur procédé d'obtention, leur application à titre de médicaments et préparation les renfermant.

(57) L'invention concerne de nouveaux polysaccharides hydro-solubles.

Ces polysaccharides extraits d'Escherichia coli et de Kleb-siella pneumoniae renferment moins de 1 % de protéines, moins de 0,5 % d'osamines, de 65 à 75 % d'oses neutres, de 18 à 27 % d'acides uroniques, de 5 à 9 % d'acide pyruvique sous forme de pyruvilidène, et ont une masse moléculaire supérieure à 50'000.

Ils possèdent une activité immunomodulatrice qui les rend utilisables dans le domaine thérapeutique.

EP 0 308 279 A1

Description

## "NOUVEAUX POLYSACCHARIDES HYDROSOLUBLES, LEUR PROCEDE D'OBTENTION, LEUR APPLICATION A TITRE DE MEDICAMENTS ET PREPARATIONS LES RENFERMANT".

La présente invention concerne de nouveaux polysaccharides immunomodulateurs, extraits d'au moins un genre bactérien de type Escherichia coli et Klebsiella pneumoniae, leur procédé d'obtention, leur application comme médicaments et les compositions les renfermant.

Certains extraits d'entérobactéries, notamment des glycoprotéines de Klebsiella pneumoniae sont connus par les demandes EP 0049.181 et 182 et 0115.988 ; et dans la demande de brevet EP.0139.551, il est décrit des procédés de préparation de principes actifs immunostimulants obtenus par l'action de bactériphages sur diverses bactéries.

Il a été apporté un perfectionnement aux procédés précédemment cités, permettant d'obtenir de nouveaux polysaccharides hydrosolubles, renfermant 65 à 75 % d'oses neutres, 18 à 27 % d'acides uroniques de 5 à 9 % d'acide pyruvique sous forme de pyruvilidène, moins de 1 % de protéines, moins de 0,5 % d'osamines et ayant une masse moléculaire supérieure à 50.000.

On désigne par oses neutres, notamment des hexoses tels que le glucose, le galactose ou le mannose ; et par acides uroniques, notamment l'acide galacturonique ; et par osamines, notamment la glucosamine.

La masse moléculaire des polysaccharides a été déterminée par la mesure du coefficient de sédimentation en ultracentrifugation analytique et par tamisage moléculaire.

Les polysaccharides de l'invention peuvent être extraits de différentes souches de bactéries ou microorganismes de façon nullement limitative ; on retient cependant tout particulièrement ceux qui proviennent des souches d'Escherichia coli et de Klebsiella pneumoniae déposées à l'Institut PASTEUR de PARIS, respectivement sous les numéros 62.23 et 58.5.

L'étude de la structure de ces polysaccharides à l'aide de différentes techniques comme la chromatographie en phase gazeuse, la spectrométrie de masse, l'électrophorèse, l'autoanalyse d'aminoacides, la chromatographie liquide à haute performance, la résonnance magnétique nucléaire de l'ultracentrifugation analytique a permis d'en préciser la composition telle que mentionnée ci-dessus. Il est à noter que la sous-fraction peptidique n'est pas responsable des activités pharmacologiques de ces polysaccharides.

La composition molaire des nouveaux polysaccharides renferme pour une molécule de galactose, de 0,2 à 0,4 molécules de glucose, de 2 à 2,5 molécules de mannose, 1 molécule d'acide galacturonique et 1 molécule d'acide pyruvique.

Les polysaccharides hydrosolubles sont isolés à partir de lysats bactériophagiques de culture d'au moins un genre bactérien de type Escherichia coli et Klebsiella pneumonia. Ces différents lysats peuvent être préparés selon une technique notamment décrite dans la demande EP. 0139.551. Et le procédé d'obtention de ces lysats du type lyse bactérienne repose sur une séquence d'au moins deux stades indispensables, à savoir une lyse bactériophagique, puis l'élimination des bactéries non lysées et des débris bactériens par filtration, centrifugation etc... Ce procédé peut être mis en oeuvre à partir d'une bactérie, ou bien avec utilisation successive de bactéries de genres différents ou encore avec utilisation simultanée de bactéries de genres différents.

Selon l'invention, on concentre un filtrat de lyse bactériophagique et procède simultanément à une sélection de molécules par passage sur membrane à perméabilité sélective, puis traite la solution de polysaccharides extraits par chromatographie, recueille la première fraction, dialyse, concentre la solution et refractionne par chromatographie, recueille la première fraction et amène à sec.

Il a été découvert qu'il était possible de perfectionner le procédé en remplaçant le milieu de culture chimiquement non défini par un milieu synthétique défini entièrement dialysable, comme les formules de milieu $M_9$ selon Adams (M.H. Adams, Bactériophages Intersciences New-York p.446, 1959) enrichi ou le milieu selon Rogers (H.J.Rogers Infect. Immuno. p.445, 1973).

Ce milieu peut alors être avantageusement éliminé par l'utilisation de dispositifs à perméabilité sélective de molécules. L'utilisation de ces mêmes dispositifs permet une concentration des filtrats de lyse et facilite le travail avec de moindres volumes. L'ultrafiltrat peut être amené à sec par exemple par lyophilisation.

La technique de sélection de molécules permet d'éliminer le milieu de culture, en particulier par utilisation de membranes à perméabilité sélective, notamment des ultrafiltres, de même la concentration des filtrats de lyse est réalisée également par la même méthode. Les membranes à perméabilité sélective peuvent être de natures diverses, par exemple en acétate de cellulose, à base de polysulfones, de polymères aromatiques, de copolymères de chlorure de vinyle et d'acrylonitrile, à base de polysulfones sur support de polyéthylène. Ces membranes peuvent se présenter sous forme plane, tubulaire ou encore sous forme de fibres creuses ou de spirales. Les membranes per mettent une concentration des molécules de masse moléculaire supérieure ou égale à 10'000 et ainsi une élimination par dialyse du milieu de culture des molécules de faible masse moléculaire. Il est souhaitable d'obtenir une concentration d'environ trente fois.

Selon une variante, on peut procéder à une phase de purification préliminaire par traitement de la solution obtenue avec un alcanol de faible masse moléculaire tel que l'éthanol, le méthanol ou l'isopropanol.

Puis, le concentrat est purifié par chromatographie sur colonne. Plusieurs types de chromatographie conviennent à la mise en oeuvre du procédé en conduisant à d'excellents résultats.

Ces chromatographies peuvent être du type tamisage moléculaire en utilisant des passages successifs sur des gels permettant l'exclusion dans un premier stade des molécules de faible masse moléculaire et, dans un deuxième stade, la séparation des molécules de haute masse moléculaire.

Ces chromatographies peuvent être de type chromatographie d'affinité en utilisant des lectines insolubilisées sur gel ou des anticorps de type IgG ou IgM insolubilisés sur gel. Les anticorps peuvent provenir du sérum d'animaux vertébrés immunisés contre les polysaccharides recherchés, notamment du lapin, du rat, du cobaye et de la souris. Ces anticorps peuvent être d'origine polyclonale ou monoclonale et produits selon les procédés bien connus du spécialiste. Les lectines et les anticorps sont insolubilisés sur des gels inertes de type agarose ou de type polyacrylamide préalablement activés par des techniques connues,telles par exemple,celle au paranitrophényle - chloroformate.

Ces chromatographies peuvent être de type chromatographie échangeuse d'ions. Les supports utilisés peuvent être à titre d'exemples des gels de polyacrylamide ou de cellulose substitués par des groupes diéthylaminoéthyl ou des résines de polystyrène-divinylbenzène substituées.

La première séparation par chromatographie peut être avantageusement de type affinité avec des anticorps immobilisés sur gel inerte ou de type échangeuse d'ions.

La deuxième séparation par chromatographie est de préférence du type échange d'ions.

Ces diverses opérations de chromatographie sont suivies à l'aide de procédés classiques notamment par l'utilisation de la réfractométrie différentielle,la spectrophotométrie,la spectrofluorométrie ou l'analyse des hexoses neutres par la méthode au résorcinol sulfurique.

La fraction correspondant aux polysaccharides recherchés dans l'invention peut alors être dialysée ou ultrafiltrée et amenée à sec par des procédés tels que l'atomisation ou la lyophilisation.

Les polysaccharides de l'invention sont doués de propriétés pharmacologiques et thérapeutiques très intéressantes ; ils sont notamment dotés de propriétés immunomodulatrices. Ils présentent chez l'homme une excellente tolérance.

Ces nouveaux polysaccharides, en tant que médicaments trouvent, par exemple, leur emploi dans le traitement ou la prévention chez l'homme et l'animal de maladies provenant d'une dysrégulation du système immunitaire, dans la prévention ou le traitement des agressions infectieuses bactériennes, virales, fongiques ou parasitaires, ainsi qu'en rhumatologie, dans la cicatrisation des plaies et en oncologie.

Les médicaments, selon l'invention, peuvent être associés à une substance antibiotique, telles des tétracyclines ; antivirale, tels des nucléotides synthétiques interférant avec la synthèse de l'acide nucléique viral ; antifongique, tel le buclosamide ; ou antiparasitaire, telle la chloroquine.

Dans une autre forme de réalisation, les médicaments,selon l'invention, peuvent être associés à d'autres produits et compositions pharmaceutiques, tel un agent anti-tumoral (cytostatiques), un agent immuno-suppressif (ciclosporine, immunoglobulines antilymphocytaires) un agent antimitotique, tels les dérivés de l'acide folique ou anti-inflammatoire, telle l'indométhacine.

Les médicaments selon l'invention peuvent aussi être couplés à des vecteurs macromoléculaires améliorant leur biodisponibilité, leur ciblage ou diminuant leur toxicité. A titre d'exemple non limitatif, ces vecteurs peuvent être des liposomes, des anticorps, des protéines ou d'autres macromolécules.

La posologie utile est susceptible de larges variations en fonction de la composition du médicament, de l'indication prescrite, de la forme d'administration et de l'âge du sujet traité. La détermination des doses utiles est laissée à l'appréciation du médecin ou du vétérinaire, comme il est de pratique habituelle, dans les traitements de modulations de la réponse immune.

A titre d'exemple, la posologie peut comporter des doses allant de 50 µg/jour à 50 mg/jour par voie orale chez l'homme dans la prévention ou la prophylaxie des infections ou complications infec tieuses postopératoires, dans les traitements des infections chroniques ou récidivantes des voies respiratoires ou urinaires. La dose usuelle par voie parentérale peut être,suivant le sujet traité et l'affection en cause, de 50 µg à 50 mg/jour chez l'homme.

A titre de médicament, les polysaccharides de l'invention peuvent être administrés par voie orale, perlinguale, parentérale, aérienne, trans-cutanée, percutanée ou topique.

Les compositions pharmaceutiques correspondantes peuvent être solides ou liquides et se présenter sous les formes pharmaceutiques couramment utilisées en médecine humaine ou vétérinaire comme par exemple : les comprimés simples ou dragéifiés, les comprimés retard, les gélules, les granulés, les poudres, les solutions, les sirops, les suppositoires, les préparations injectables lyophilisées ou non, les ovules, les crèmes, les gels, les pommades, les lotions, les gouttes, les collyres, les aérosols. Les principes actifs peuvent être incorporés à des excipients habituellement employés dans ces compositions pharmaceutiques, tels que le talc, le stéarate de magnésium, la silice colloïdale, le saccharose, le sorbitol, le mannitol, les véhicules aqueux ou non, les corps gras d'origine animale ou végétale, les dérivés paraffiniques, les glycols, les divers agents mouillant ou émulsifiant, les conservateurs.

Il va être donné maintenant, à titre non limitatif, des exemples de mise en oeuvre de l'invention.

Exemple 1

Préparation avec utilisation successive des bactéries de genre Klebsiella et Escherichia.

La bactérie du genre Klebsiella, espèce pneumoniae, est de sérotype K68 répertoriée à l'Institut Pasteur de Paris sous la référence 58,5.

La bactérie du genre Escherichia, espèce coli, est de sérotype 0119 : B14, répertoriée par l'Institut Pasteur de Paris sous la référence 62.23.

Le phage, homologue de Klebsiella, appartient au groupe morphologique $C_1$ (nomenclature du Sous-Comité de Taxonomie des Bactériophages de l'International Committee for Taxonomy of Virus de l'I.A.M.S.), il a une tête icosaédrique, une queue courte, difficile à mettre en évidence, le manchon et la plaque terminale ne sont pas détectables. Sur milieu gélosé, les plages de lyse ont de 0,5 à 3mm et sont de forme très irrégulière, avec des contours estompés et un cen tre clair. Les clones [r+] sont rares.

Le coliphage appartient au groupe morphologique $C_1$ (nomenclature du sous-comité de Taxonomie des Bactériophages de l'International Committee for Taxonomy of Virus de l'I.A.M.S.), il a une tête isométrique, une queue très courte, difficile à mettre en évidence, un manchon et une plaque terminale non détectables. Sur milieu gélosé, les plages de lyse ont un diamètre de 1 à 1,5mm, une forme ronde, des contours réguliers, un centre voilé et des clones [r+] confluents.

Les deux souches bactériennes sont cultivées au moyen d'un fermenteur dans un milieu synthétique entièrement dialysable de composition:

$NH_4Cl$    1,00 g
$KH_2PO_4$    3,00 g
$Na_2PO_412H_2O$    6,00 g
$MgSO_47H_2O$    4,55 g
D-Glucose    140,00 g
L-Leucine    78,00mg
L-Histidine
HCl    60,00mg
L-Isoleucine    78,00mg
L-Valine    67,00mg
L-Tryptophane    15,00mg
L-Arginine    180,00 mg
L-Glutamine    417,00 mg
L-Lysine HCl    104,00 mg
L-Méthionine    22,00 mg
L-Phénylalanine    47,00 mg
L-Thréonine    68,00 mg
Acide folique    0,50 mg
Inositol    1,00 mg
Nicotinamide    0,50 mg
Pyridoxal HCl    0,50 mg
Panthoténate de Ca    0,50 mg
Chlorure de Choline    0,50 mg
Thiamine HCl    0,50 mg

Le milieu est ensemencé avec un inoculum de Klebsiella pneumoniae qui se trouve en phase de croissance stationnaire. La densité initiale est de $1.10^7$ bactéries par ml. La culture est placée à 37°C, sous agitation modérée.

A la fin de la phase de latence, soit environ 60 minutes après l'ensemencement, les bactéries sont infectées par leurs phages homologues, la multiplicité d'infection optimale est de 0,03.

L'évolution de la lyse est suivie à l'aide d'un compteur cellulaire. Lorsqu'il reste moins de 10 bactéries par ml, le milieu est à nouveau ensemencé avec un inoculum d'Escherichia qui se trouve en phase de croissance stationnaire. La densité initiale de cette nouvelle culture est de $5.10^7$ Escherichia par ml.

A la fin de la phase de latence,soit dans ces conditions expérimentales, 20 minutes après le deuxième ensemencement, les bactéries sont infectées par les coliphages. La multiplicité d'infection est optimalement de 0,1.

L'évolution de la lyse est suivie à l'aide d'un compteur cellulaire. Lorsqu'il reste moins de $10^3$ Escherichia par ml, la culture est stoppée par passage à travers un filtre membrane stérilisant de 0,22 µm.

Le filtrat est ensuite dialysé et concentré par ultrafiltration tangentielle. La solution résultante peut être amenée à sec par lyophilisation ou passée directement sur une colonne de 5cm, de diamètre renfermant 1 litre de DEAE Trisacryl ®. L'évolution se fait par un gradient d'une solution de phosphate de sodium dont la concentration varie de 50 à 650mM. Le pH de cette solution est de 7,5. Les fractions correspondant au premier pic d'élution (détection des hexoses neutres par la méthode au résorcinol sulfurique) sont rassemblées et dialysées contre une solution de phosphate de sodium à 10 mM et pH 7,5. Le fraction obtenue est alors passée sur une colonne de 2,5 cm de diamètre renfermant 500 ml de résine DOWEX 50-X2 ® de 100 à 200 mesh (éluant : eau distillée).

Les fractions correspondant au premier pic d'élution (détection par réfractométrie ou des hexoses neutres par la méthode au résorcinol) sont neutralisées par la soude IN ou de l'ammoniaque à 5 %, rassemblées et lyophilisées. On obtient les polysaccharides recherchés purifiés,dont la composition molaire est la suivante : pour une molécule de galactose, 0,2 molécule de glucose, 2,2 molécules de mannose, 1 molécule d'acide galacturonique, 1 molécule d'acide pyruvique sous forme de pyruvilidène.

Exemple 2 :

Les polysaccharides sont obtenus selon le mode opératoire de l'exemple 1 en n'utilisant que la bactérie de souche Klebsiella pneumoniae. La composition des polysaccharides obtenus est la suivante : 69 % d'hexoses neutres, 0,25 % de glucosamine, 7 % d'acide pyruvique, 22,7 % d'acide galacturonique et 0,86 % de protéines.

Exemple 3 :

Le filtrat de lyse des souches bactériennes de Escherichia coli et Klebsiella pneumoniae, obtenu selon la procédure de l'exemple 1, est dialysé contre un tampon de phosphate de sodium à 150 mM et pH 7,4. Le filtrat est ensuite passé sur une colonne de 5cm de diamètre contenant 500 ml de gel GF 2000 Trisacryl ® sur lequel est fixé de façon covalente 1,5 mg d'anticorps spécifiques par ml de gel. Après 1 heure d'incubation, la colonne est lavée par 4 fois son volume d'un tampon phosphate 150 mM à pH 7,4. L'élution se fait par une solution du même tampon contenant 2 M de chlorure de magnésium. Une fraction égale à 2 fois le volume de la colonne est retenue et dialysée contre de l'eau distillée. Cette fraction est lyophilisée et contient les polysaccharides de composition molaire suivante : pour 1 molécule de galactose, 2,5 molécules de mannose, 0,3 molécule de glucose, 1 molécule d'acide galacturonique, 1 molécule d'acide pyruvique sous forme de pyruvilidène.

ETUDES PHARMACOLOGIQUES

Les activités sur l'animal et sur l'homme des polysaccharides de l'invention ont été testées avec les produits obtenus selon les exemples 1 et 2.

Activation des macrophages alvéolaires murins

Ce test met en évidence le phénomène d'activation des macrophages par une mesure de leur activité cytostatique ou cytotoxique envers des cellules de lignée tumorale.

Les leucocytes phagocytaires de la classe des monocytes-macrophages peuvent subir un processus d'activation qui se manifeste par des propriétés telles que l'augmentation de la phagocytose, l'augmentation de la sécrétion d'enzymes protéolytiques, de métabolites de l'oxygène tel que l'anion superoxyde ou le peroxyde d'hydrogène et de facteurs cytotoxiques.

Ces processus contribuent à accroître le pouvoir bactéricide et tumoricide de ces cellules.

De plus, ce processus d'activation permet à ces cellules d'augmenter leur capacité de présentation de divers antigènes aux lymphocytes T coopérants. Enfin, le phénomène d'activation des macrophages permet la sécrétion de monokines telles les interférons ou l'interleukine 1 qui interviennent dans le processus de réponse immunitaire.

Le test de mesure de l'activation des macrophages consiste à traiter une culture de macrophages alvéolaires ou péritonéaux de rat ou de souris ou de monocytes humaines par des doses des polysaccharides de l'invention variant entre 0,001 et 50 µg/ml. Il est réalisé de la manière suivante :

$10^5$ macrophages de souris de souche Balb c, Swiss, C57/B16 ou DBA/2 ou de rat de souche Kyoto ou Wistar sont placés dans une plaque de microtitration dans du milieu de culture complété ou non par du sérum artificiel et de 1 à 10 µg/ml de polymyxine.

On ajoute les polysaccharides de l'invention ou le tampon correspondant sans polysaccharides comme contrôle négatif. Les cellules sont incubées 24 heures à 37°C. On élimine ensuite le surnageant et lave les cellules avec du milieu de culture.

On ajoute alors les cellules cibles tumorales L1210 ou P815 dans du milieu complet. Le rapport macrophages/cellules cibles est de 10. On incube alors la culture durant 24 heures à 37°C. Puis on ajoute 1 - 2 x $10^4$Bq de thymidine tritiée par puits. Les cellules sont récupérées après 4 heures d'incubation à 37°C sur un filtre en fibre de verre. Après séchage des filtres, la radioactivité est mesurée en présence d'un mélange scintillant.

Les résultats correspondent à une moyenne de 3 mesures. Le pourcentage d'activation est exprimé :

$$A \% = \frac{R - S}{R} \times 100$$

R : radioactivité incorporée par les cellules tumorales cultivées en présence de macrophages de référence.

S : radioactivité incorporée par les cellules tumorales cultivées en présence de macrophages stimulés.

Les résultats consignés dans le tableau I montrent l'activité des produits selon l'exemple 1 et 3.

TABLEAU I

| Produit testé | Dose | % d'activation des macrophages alvéolaires de rats Kyoto. |
|---|---|---|
| Polysaccha-rides de ex 1 ex 3 | 0,01 µg/ml 0,1 µg/ml 1 µg/ml 10 µg/ml | 58 % 62 % 60 % 86 % |
| Muramyl dipeptide (MDP) référence positive | 0,01 µg/ml 1 µg/ml 10 µg/ml | 28 % 90 % 92 % |
| Placebo | | 0 % |

Activité inductrice d'interféron invivo chez l'homme

Les interférons sont un groupe de molécules d'origine protéique présentant des propriétés antivirales et immunomodulatrices. Dans des conditions normales, la clairance rapide de l'interféron sérique ne permet pas un dosage direct dans le sérum humain. Il est possible, pour surmonter cette difficulté, de mesurer l'activité d'une enzyme induite par l'interféron : la 2'-5' oligo-isoadenylate synthétase. On a pu démontrer que cette enzyme est un bon marqueur de l'interféron (réf. L.J.Z.Penn et B.R.G.Williams, J.Virol., 49, 1984, p.748-753) chez les sujets sains, son activité dans les lymphocytes du sang périphérique est constante. Par contre, celle-ci augmente fortement en cas d'infection virale ou lors d'administration exogène d'interféron.

L'effet d'une administration orale unique en dose élevée des polysaccharides de l'invention a été testée chez 10 volontaires.

Un groupe de 5 individus a reçu un placebo alors que l'autre groupe de 5 individus a reçu une dose unique de 2 mg des polysaccharides de l'invention préparés selon l'exemple 1.

Des prises de sang ont été effectuées avant l'administration du produit ainsi que 24 et 48 heures après. Les leucocytes périphériques ont été isolés et lysés. L'activité de la 2'-5' oligo-isoadenylate synthétase a été mesurée dans ces lysats.

Les résultats reproduits dans le tableau II démontrent qu'une dose unique de 2 mg de produits induisent une augmentation significative de l'activité de la 2'-5' oligo-isoadenylate synthétase particulièrement 48 heures après la prise orale.

TABLEAU II

Variation de l'activité de la 2'-5' oligo-isoadenylate synthétase. Les valeurs initiales ont été rapportées à 100 %.

| | 0 h | 24 h | 48 h |
|---|---|---|---|
| Placebo | 100 | 82 | 109 |
| 2mg produit de l'exemple 1 | 100 | 145 | 208 |

Induction de facteurs inhibiteurs de la migration des leucocytes (MIF-LIF)

Une étude contre placebo a été réalisée chez 2 groupes de volontaires recevant un placebo ou une dose unique du produit selon l'exemple 1 par voie orale à la dose unique de 2 mg. L'administration est réalisée de façon randomisée en double aveugle.

**EP 0 308 279 A1**

Des prises de sang sont effectuées 0 h, 24h, 48h, 72h et 96h après la prise du produit.

Les résultats décrits dans le tableau III démontrent clairement que le produit induit une inhibition significative de la migration des leucocytes par rapport au placebo.

TABLEAU III

|  | 0 h | 24 h | 48 h | 96 h |
|---|---|---|---|---|
| Placebo | 0 | 0 | 3 | 3 |
| Produit de l'exemple 1 2mg | 0 | 8 | 22 | 28 |

Les résultats sont exprimés en % d'inhibition de la migration.

L'induction de tels facteurs solubles démontre l'effet des polysaccharides de l'invention sur les mécanismes effecteurs de la réponse immune.

L'intérêt de l'induction de ces facteurs est illustré par les essais cliniques rapportés ci-dessous.

Prévention et traitement des infections chroniques ou récidivante des voies respiratoires.

Trois expérimentateurs ont étudié sur le plan clinique les polysaccharides de l'invention selon l'exemple 1 chez une population de patients atteint de bronchite chronique. Ces patients ont présenté au moins 2 épisodes infectieux dans les 12 mois précédant l'essai. Ont été exclus les patients présentant des troubles graves des fonctions hépatique, rénale ou cardiaque. De même ceux recevant une antibiothérapie systématique, un traitement immunomodulateur ou une corticothérapie.

46 patients entrant dans ces critères ont été traités par une prise orale de 0,06 mg de polysaccharides ou d'un placebo à jeun le matin, 20 jours consécutifs par mois durant 3 mois.

L'étude a été menée en double aveugle. Les patients ont été observés durant les 9 mois qui ont suivi le début du traitement.

Les résultats consignés dans le tableau IV montrent l'effet du produit durant les 3 mois suivant le traitement.

TABLEAU IV

|  | Produit de l'invention Ex.1 | Placebo | % de diminution | signification statistique |
|---|---|---|---|---|
| Nombre d'épisodes infectieux | 0,57 | 0,91 | 37,4 % | $p < 0,05$ |
| Durée des épisodes infectieux (jours) | 5,91 | 11,96 | 50,6 % | $p < 0,05$ |
| Durée de l'antibiothérapie(jours) | 6,04 | 10,17 | 40,6 % | $p < 0,05$ |

Ces résultats indiquent clairement une diminution significative des épisodes infectieux, de la durée de ces épisodes et de l'antibiothérapie chez les patients traités par les polysaccharides de l'invention, selon l'exemple 1.

Prévention des complications infectieuses post-opératoires

La dépression de l'immunité à médiation cellulaire (DTH) consécutive à un choc opératoire est un phénomène bien connu. Trois études ont eu pour objet l'effet d'un traitement oral avec les polysaccharides de l'exemple 1 sur l'incidence des complications infectieuses post-opératoires.

58 patients furent traités durant la période de 7 jours précédant et suivant l'intervention chirurgicale. Les traitements ont été aministrés par voie orale à raison de 0,06 mg par jour selon une méthodologie en double-aveugle randomisée. L'immunité à médiation cellulaire a été évaluée 7 jours avant l'intervention, le jour de l'intervention et 7 jours après par la mesure de la réaction cutanée obtenue en réponse à 7 antigènes entrainant une réponse immunitaire cellulaire (système IMC-test de Biomérieux).

Les résultats obtenus sont répertoriés dans le tableau V.

7

EP 0 308 279 A1

TABLEAU V

|  | Polysaccha-rides de l'exemple 1 | Placebo |
|---|---|---|
| Différence du score IMC-TEST entre J-7 et J0 | - 6,3 mm | - 11,9 mm |
| Signification statistique | p < 0,01 | p < 0,001 |

La dépression de l'immunité à médiation cellulaire provoquée par l'intervention chirurgicale est nettement moins grande chez les patients traités avec le produit que chez ceux sous placebo. D'autre part, durant les 7 jours suivant l'acte chirurgical, le produit de l'invention entraine une récupération plus rapide des valeurs normales de l'immunité cellulaire par rapport au placebo.

Le risque infectieux étant directment proportionnel au score de l'immunité cellulaire (réf. : J.I. GALLIN and A.S. FAUCI; Advances in Host Defense Mechanisms. Ed. Raven Press, New York, 1986, vol. 6), le produit de l'invention peut être considéré comme efficace dans la prévention et le traitement des complications infectieuses post-opératoires.

ETUDES TOXICOLOGIQUES

Toxicité aigüe

Aucune toxicité des polysaccharides de l'invention n'a été observée (mortalité, aspect macroscopique des organes) chez le rat après une administration orale de 20'000 fois la dose thérapeutique.

Toxicité chronique

Aucune toxicité chronique durant 4 mois de traitement chez le rat et le chien pour une dose journalière orale des polysaccharides de l'invention allant jusqu'à 1'800 fois la dose thérapeutique.

Mutagénicité

Les tests bactériens de Ames ont démontré une absence de pouvoir mutagène des polysaccharides de l'invention.

TOLERANCE

Les polysaccharides de l'invention administrés durant 3 mois consécutifs à la dose thérapeutique de 0,06 mg/jour sont parfaitement bien tolérés. Les examens de tolérance biologique réalisés lors des différents essais cliniques n'ont démontré aucune variation anormale des paramètres suivants : formule sanguine, hémoglobine, électrolytes, urée, glycémie, protéines sériques, créatinine, acide urique, cholestérol total, ALAT, ASAT, CK, phosphatases alcalines et bilirubine. La tolérance est jugée par les praticiens et les patients comme excellente.

**Revendications**

1. Polysaccharides extraits d'au moins un genre bactérien de type Escherichia coli et Klebsiella pneumoniae, caractérisée en ce qu'ils renferment 65 à 75 % d'oses neutres, 18 à 27 % d'acides uroniques, de 5 à 9 % d'acide pyruvique sous forme de pyruvilidène, moins de 0,5 % d'osamines, moins de 1 % de protéines, et ayant une masse moléculaire supérieure à 50'000.

2. Polysaccharides selon la revendication 1, caractérisés en ce qu'il renferment 65 à 75 % d'hexoses tels que le glucose, le galactose ou le mannose, 18 à 27 % d'acide uronique tel que l'acide galacturonique, 5 à 9 % d'acide pyruvique, moins de 1 % de protéines, et moins de 0,5 % de glucosamine.

3. Polysaccharides selon la revendication 1 ou 2, caractérisés en ce qu'ils renferment pour une molécule de galactose, de 0,2 à 0,4 molécule de glucose, 2 à 2,5 molécules de mannose, une molécule d'acide galacturonique et une molécule d'acide pyruvique.

4. Polysaccharides selon la revendication 1 ou 2, caractérisés en ce qu'ils sont extraits d'au moins une souche choisie parmi les souches Klebsiella pneumoniae K 68, Escherichia coli 0119 déposées à l'Institut Pasteur sous les références respectives 58.5 et 62.23.

5. Procédé d'obtention de polysaccharides selon une quelconque des revendications 1 à 4, à partir

8

d'un filtrat de lyse bactériophagique de culture d'au moins un genre bactérien de type Escherichia coli ou Klebsiella pneumoniae, caractérisé en ce qu'on concentre le dit filtrat et procède simultanément à une sélection de molécules par passage sur membranes à perméabilité sélective, puis traite la solution de polysaccharides extraits par chromatographie, recueille la première fraction, dialyse, concentre la solution et refractionne par chromatographie, recueille la première fraction et amène à sec.

6. Procédé d'obtention de polysaccharides selon la revendication 5, caractérisé en ce que la première chromatographie est de type affinité avec des anticorps immobilisés ou de type échange d'ions, la deuxième chromatographie est de type échange d'ions, et la fraction contenant les polysaccharides est amenée à sec par lyophilisation.

7. Procédé d'obtention de polysaccharides selon la revendication 5 ou 6, caractérisé en ce que le filtrat de lyse bactérienne est obtenu avec utilisation simultanée de bactéries des genres Escherichia coli et Klebsiella pneumoniae.

8. Médicament caractérisé en ce que le principe actif est constitué par les polysaccharides selon une quelconque des revendications 1 à 4, associé à un excipient adapté au type d'administration.

9. Médicament selon la revendication 8, caractérisé en ce qu'il comporte en outre une substance antibiotique, antivirale, antifongique ou antiparasitaire.

10. Médicament selon la revendication 8, caractérisée en ce qu'il comporte en outre un agent anti-tumoral, immuno-suppressif, anti-mitotique ou anti-inflammatoire.

11. Médicament selon la revendication 8, caractérisé en ce que le principe est couplé à un vecteur macromoléculaire.

**Revendications pour l'Etat contractant suivant: GR**

1. Procédé d'obtention de nouveaux polysaccharides à partir d'un filtrat de lyse bactériophagique de culture d'au moins un genre bactérien de type Escherichia coli ou Klebsiella pneumoniae, caractérisé en ce qu'on concentre ledit filtrat et procède simultanément à une sélection de molécules par passage sur membranes à perméabilité sélective, puis traite la solution de polysaccharides extraits par chromatographie, recueille la première fraction, dialyse, concentre la solution et refractionne par chromatographie, recueille la première fraction et amène à sec.

2. Procédé d'obtention de nouveaux polysaccharides selon la revendication 1, caractérisé en ce que la première chromatographie est de type affinité avec des anticorps immobilisés ou de type échange d'ions, la deuxième chromatographie est de type échange d'ions, et la fraction contenant les polysaccharides est amenée à sec par lyophilisation.

3. Procédé d'obtention de nouveaux polysaccharides selon la revendication 1 ou 2, caractérisé en ce que le filtrat de lyse bactérienne est obtenu avec utilisation simultanée de bactéries des genres Escherichia coli et Klebsiella pneumoniae.

4. Procédé d'obtention de nouveaux polysaccharides selon une quelconque des revendications 1 à 3, caractérisé en ce que les dits polysaccharides renferment 65 à 75 % d'oses neutres, 18 à 27 % d'acides uroniques, de 5 à 9 % d'acide pyruvique sous forme de pyruvilidène, moins de 0,5 % d'osamines, moins de 1 % de protéines, et ayant une masse moléculaire supérieure à 50'000.

5. Procédé d'obtention de nouveaux polysaccharides selon la revendication 4, caractérisé en ce qu'il renferme 65 à 75 % d'hexoses tels que le glucose, le galactose ou le mannose, 18 à 27 % d'acide uronique tel que l'acide galacturonique, 5 à 9 % d'acide pyruvique, moins de 1 de protéines, et moins de 0,5 % de glucosamine.

6. Procédé d'obtention de nouveaux polysaccharides selon la revendication 5, caractérisé en ce qu'il renferme pour une molécule de galactose, de 0,2 à 0,4 molécule de glucose, 2 à 2,5 molécules de mannose, une molécule d'acide galacturonique et une molécule d'acide pyruvique.

7.Procédé d'obtention de nouveaux polysaccharides selon la revendication 1, caractérisés en ce qu'ils sont extraits d'au moins une souche choisie parmi les souches Klebsiella pneumoniae K 68, Escherichia coli 0119 déposées à l'Institut Pasteur sous les références respectives 58.5 et 62.23.

**Revendications pour l'Etat contractant suivant: ES**

1. Procédé d'obtention de nouveaux polysaccharides à partir d'un filtrat de lyse bactériophagique de culture d'au moins un genre bactérien de type Escherichia coli ou Klebsiella pneumoniae, caractérisé en ce qu'on concentre ledit filtrat et procède simultanément à une sélection de molécules par passage sur membranes à perméabilité sélective, puis traite la solution de polysaccharides extraits par chromatographie, recueille la première fraction, dialyse, concentre la solution et refractionne par chromatographie, recueille la première fraction et amène à sec.

2. Procédé d'obtention de nouveaux polysaccharides selon la revendication 1, caractérisé en ce que la première chromatographie est de type affinité avec des anticorps immobilisés ou de type échange d'ions, la deuxième chromatographie est de type échange d'ions, et la fraction contenant les polysaccharides est amenée à sec par lyophilisation.

3. Procédé d'obtention de nouveaux polysaccharides selon la revendication 1 ou 2, caractérisé en ce que le filtrat de lyse bactérienne est obtenu avec utilisation simultanée de bactéries des genres Escherichia coli et Klebsiella pneumoniae.

4. Procédé d'obtention de nouveaux polysaccharides selon une quelconque des revendications 1 à 3, caractérisé en ce que les dits polysaccharides renferment 65 à 75 % d'oses neutres, 18 à 27 % d'acides uroniques, de 5 à 9 % d'acide pyruvique sous forme de pyruvilidène, moins de 0,5 % d'osamines, moins de 1 % de protéines, et ayant une masse moléculaire supérieure à 50'000.

5. Procédé d'obtention de nouveaux polysaccharides selon la revendication 4, caractérisé en ce qu'il renferme 65 à 75 % d'hexcses tels que le glucose, le galactose ou le mannose, 18 à 27 % d'acide uronique tel que l'acide galacturonique, 5 à 9 % d'acide pyruvique, moins de 1 de protéines, et moins de 0,5 % de glucosamine.

6. Procédé d'obtention de nouveaux polysaccharides selon la revendication 5, caractérisé en ce qu'il renferme pour une molécule de galactose, de 0,2 à 0,4 molécule de glucose, 2 à 2,5 molécules de mannose, une molécule d'acide galacturonique et une molécule d'acide pyruvique.

7.Procédé d'obtention de nouveaux polysaccharides selon la revendication 1, caractérisés en ce qu'ils sont extraits d'au moins une souche choisie parmi les souches Klebsiella pneumoniae K 68, Escherichia coli 0119 déposées à l'Institut Pasteur sous les références respectives 58.5 et 62.23.

Office européen
des brevets

RAPPORT DE RECHERCHE EUROPEENNE

Numero de la demande

EP  88 40 2048

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | CARBOHYDRATE RESEARCH, vol. 86, novembre 1980, pages 259-271, Elsevier Scientific Publishing Co., Amsterdam, NL; K. OKUTANI et al.: "Structural investigation of Klebsiella serotype K46 polysaccharide" * Page 262 * --- | 1-3 | C 12 P 19/04 C 08 B 37/00 A 61 K 31/715// (C 12 P 19/04 C 12 R 1:19 C 12 R 1:22 ) |
| A | GB-A-2 168 365  (ROUSSEL-UCLAF) * Résumé * --- | 1-3 | |
| D,A | EP-A-0 139 551  (LIPHA) * Résumé; exemple 3 * ----- | 1-11 | |

|  |  |  | DOMAINES TECHNIQUES RECHERCHES (Int. Cl.4) |
|---|---|---|---|
|  |  |  | C 12 P C 08 B |

Le présent rapport a été établi pour toutes les revendications

| Lieu de la recherche | Date d'achèvement de la recherche | Examinateur |
|---|---|---|
| LA HAYE | 05-10-1988 | SOMERVILLE F.M. |